# EUROPEAN PATENT APPLICATION

(11) **EP 4 201 435 A1**
(43) Date of publication of application: **28.06.2023**
(21) Application number: 21858250.0
(22) Date of filing: 12.08.2021
(51) Int. Cl.: A61L 9/22, A61L 9/015, A61L 9/14, A61L 9/20, C12M 1/12

(54) **ANTIMICROBIAL METHOD AND ANTIMICROBIAL DEVICE**

(30) Priority: 19.08.2020 JP 2020138768
(71) Applicant: Sharp Kabushiki Kaisha, Sakai-shi Osaka 590-8522 (JP)
(72) Inventor: YAMAMOTO Satohiko, Sakai City, Osaka 5908522 (JP)
(74) Representative: Treeby, Philip David William
(86) International application number: PCT/JP2021/029755
(87) International publication number: WO 2022/039103

(57) **Abstract**

An antimicrobial device (50) includes an antimicrobial unit (60), an ion unit (70), and a control unit (80). The antimicrobial unit (60) delivers at least one of light having an antimicrobial effect, a gas having an antimicrobial effect, or liquid particles having an antimicrobial effect toward a target space (14). The ion unit (70) delivers ions toward the target space (14). The control unit (80) controls the antimicrobial unit (60) and the ion unit (70). Each of the antimicrobial effect of the light, the antimicrobial effect of the gas, and the antimicrobial effect of the liquid particles is stronger than an antimicrobial effect of the ions.

## Description

### Technical Field

The present invention relates to an antimicrobial method and an antimicrobial device.

### Background Art

When cells are cultured, the work is performed in a clean space (sterile space) to inhibit contamination. PTL 1 discloses an example of a safety cabinet that forms a clean space.

### Citation List

### Patent Literature

PTL 1: JP 2016-165249 A

### Summary of Invention

### Technical Problem

To maintain a clean space, sterilization treatments are performed at an appropriate timing for the cell culture. Sterilization treatments are typically performed by using formalin, a peracetic acid preparation, hydrogen peroxide, ultraviolet rays, or the like. However, because such sterilization treatments are harmful to a human body, it must be ensured that there is no residue of the substance used for the sterilization treatment before a worker enters the clean space after the sterilization treatment. Contamination can occur in a clean space during time to ensure no residue of the substance, when a worker enters the clean space, or the like.

The present invention is made in view of the problems described above, and an object thereof is to provide an antimicrobial method and an antimicrobial device that can favorably maintain a hygienic environment in a target space.

### Solution to Problem

According to a first aspect of the present invention, an antimicrobial method includes at least two antimicrobial treatment processes, and an ion treatment process. In the antimicrobial treatment process, at least one of light having an antimicrobial effect, a gas having an antimicrobial effect, or liquid particles having an antimicrobial effect is delivered toward a target space. In the ion treatment process, ions are delivered toward the target space. A start time of the ion treatment process is included in a period from a start time of a first instance of the antimicrobial treatment process to before a start time of a second instance of the antimicrobial treatment process. An end time of the ion treatment process is after an end time of the first instance of the antimicrobial treatment process.

According to a second aspect of the present invention, an antimicrobial device includes an antimicrobial unit, an ion unit, and a control unit. The antimicrobial unit delivers at least one of light having an antimicrobial effect, a gas having an antimicrobial effect, or liquid particles having an antimicrobial effect toward a target space. The ion unit delivers ions toward the target space. The control unit controls the antimicrobial unit and the ion unit.

### Advantageous Effects of Invention

According to an antimicrobial method and an antimicrobial device of the present invention, it is possible to favorably maintain a hygienic environment in a target space.

### Brief Description of Drawings

FIG. 1 is a diagram illustrating a configuration of an antimicrobial system according to a first embodiment of the present invention.
FIG. 2 is a flowchart illustrating an antimicrobial method according to the first embodiment.
FIG. 3 is a diagram illustrating the configuration of the antimicrobial system according to the first embodiment.
FIG. 4 is a timetable illustrating the antimicrobial method according to the first embodiment.
FIG. 5 is a flowchart illustrating the antimicrobial method according to the first embodiment.
FIG. 6 is a diagram illustrating a configuration of an antimicrobial system according to a second embodiment of the present invention.
FIG. 7 is a diagram illustrating a configuration of an antimicrobial system according to a third embodiment of the present invention.
FIG. 8 is a diagram illustrating a configuration of an antimicrobial system according to a fourth embodiment of the present invention.

### Description of Embodiments

Embodiments of the present invention will be described hereinafter with reference to the accompanying drawings. Note that, in the drawings, the same or equivalent components are denoted by the same reference numerals and signs, and description thereof will not be repeated.

### First Embodiment

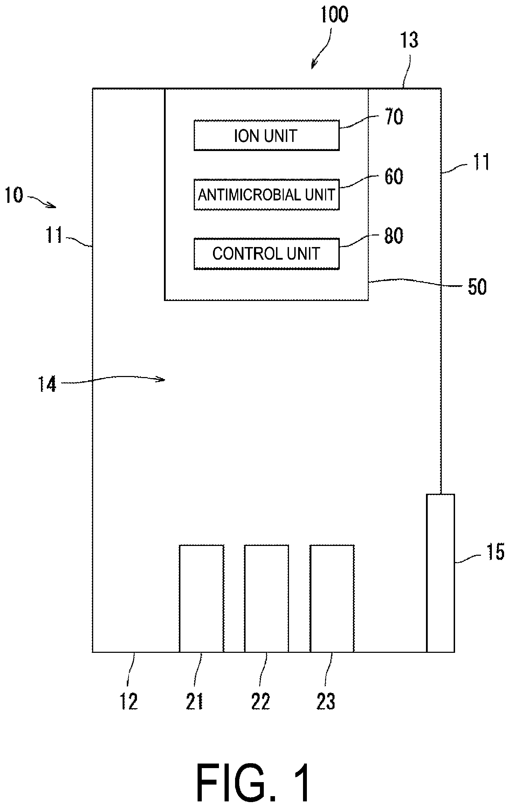

An antimicrobial system 100 will be described with reference to FIG. 1. FIG. 1 is a diagram illustrating a configuration of the antimicrobial system 100 according to a first embodiment of the present invention. As illustrated in FIG. 1, the antimicrobial system 100 is, for example, a cell culture facility for culturing a cell of a multicellular organism related to regenerative medicine and for workers to perform cell culture related work. The cell is, for example, a cell derived from an animal and a plant and, in the first embodiment, is an induced pluripotent stem cell (iPS cell). Note that the antimicrobial system 100 may be used as a system for culturing a cell other than a cell for the regenerative medicine.

Specifically, the antimicrobial system 100 includes a workroom 10 and an antimicrobial device 50. Note that the antimicrobial system 100 may include a plurality of the workrooms 10. The antimicrobial system 100 may also include a plurality of the antimicrobial devices 50.

The workroom 10 includes a plurality of wall surfaces 11, a floor 12, and a ceiling 13. A workspace 14 is defined by the plurality of wall surfaces 11, the floor 12, and the ceiling 13. The workspace 14 is an example of a target space. The workspace 14 is a space for culturing cells and performing cell culture related work. A door 15 is provided on at least one wall surface 11 of the plurality of wall surfaces 11. A worker enters and exits the workspace 14 by the door 15 being opened and closed.

A safety cabinet 21, a clean chamber 22, and an incubator 23, for example, are disposed in the workspace 14. Note that the arrangement of the safety cabinet 21, the clean chamber 22, and the incubator 23 is not particularly limited, and only a part of the safety cabinet 21, the clean chamber 22, and the incubator 23 may be disposed.

The antimicrobial device 50 includes an antimicrobial unit 60, an ion unit 70, and a control unit 80.

The antimicrobial device 50 is disposed in at least one location of the plurality of wall surfaces 11, the floor 12, or the ceiling 13. Specifically, the antimicrobial device 50 is disposed in the ceiling 13.

The antimicrobial unit 60 delivers at least one of light having an antimicrobial effect, a gas having an antimicrobial effect, or liquid particles having an antimicrobial effect toward the workspace 14. In other words, the antimicrobial unit 60 executes an antimicrobial treatment process. In this description, the concept of "antimicrobial" includes not only the killing of microorganisms such as bacteria, molds, and viruses, for example, but also the inhibiting of growth and proliferation of the microorganisms.

Specifically, the antimicrobial unit 60 includes an ozone generation device and a delivery unit. The ozone generation device is, for example, an electric discharge device that generates ozone (O₃) from oxygen molecules (O₂) by electric discharge. Ozone (O₃) is an example of a gas having an antimicrobial effect. Specifically, the ozone generation device includes a pair of electric discharge electrodes (not illustrated). Examples of an electric discharge method include the plasma discharge or the corona discharge.

The delivery unit delivers ozone (O₃) toward the workspace 14. The delivery unit is, for example, a fan. The fan rotates. The fan generates an air current by rotating. The air current generated by the delivery unit includes ozone (O₃), and the delivery unit delivers the ozone (O₃) toward the workspace 14. Specifically, an installation position and an airflow direction of the antimicrobial unit 60 are set so that the air current generated by the delivery unit reaches every corner of the workspace 14. As a result, the ozone (O₃) diffuses throughout the workspace 14.

The ozone (O₃) surrounds a surface of the microorganism when released into the air. The ozone (O₃) can inactivate the microorganism to reduce the life activity of the microorganism. Note that ozone (O₃) is an active species having high oxidizability and thus may adversely affect not only microorganisms, but also humans and cells when released to humans and cells.

The ion unit 70 delivers ions toward the workspace 14. In other words, the ion unit 70 executes an ion treatment process.

The antimicrobial effect of ions is weaker than the antimicrobial effect of ozone (O₃). However, ions can inactivate microorganisms to reduce the life activity of the microorganisms. Furthermore, ions have little or no adverse effect on humans and cells.

Specifically, an ion generation unit is an electric discharge device that generates ions by electric discharge. More specifically, the ion generation unit is an electric discharge device that generates a positive ion and a negative ion by electric discharge. Note that the ion generation unit may be an electric discharge device that generates only one of the positive ion and the negative ion by electric discharge.

The control unit 80 controls the antimicrobial unit 60 and the ion unit 70. The control unit 80 is, for example, a computer. The control unit 80 includes a processor such as a central processing unit (CPU), for example. Specifically, the control unit 80 switches the antimicrobial unit 60 and the ion unit 70 on and off. On refers to, for example, a power source being turned on. Off refers to, for example, the power source being turned off.

Specifically, the control unit 80 turns on the antimicrobial unit 60 in a first period and turns off the antimicrobial unit 60 in periods other than the first period. In other words, the control unit 80 executes the antimicrobial treatment process in which ozone (O₃) is delivered from the antimicrobial unit 60 toward the workspace 14 during the first period. Furthermore, the control unit 80 executes at least two antimicrobial treatment processes. For example, the control unit 80 executes antimicrobial treatment processes at a predetermined interval (for example, 1 day or 1 week).

Furthermore, the control unit 80 turns on the ion unit 70 in a second period and turns off the ion unit 70 in periods other than the second period. In other words, the control unit 80 executes the ion treatment process of delivering ions from the ion unit 70 toward the workspace 14 during the second period.

A part of the second period is included in a period after execution of the antimicrobial treatment process and before execution of the next antimicrobial treatment process. In other words, a part of the second period is included in a period after execution of the antimicrobial treatment process and before execution of the next antimicrobial treatment process immediately following the antimicrobial treatment process. Specifically, a start time of the ion treatment process is included in a period from a start time of the first antimicrobial treatment process to before a start time of the next antimicrobial treatment process. Specifically, the start time of the ion treatment process may be a time before an end time of the first antimicrobial treatment process, may be the same time as the end time of the first antimicrobial treatment process, or may be a time after the end time of the first antimicrobial treatment process. Furthermore, an end time of the ion treatment process is a time after the end time of the first antimicrobial treatment process. Specifically, the end time of the ion treatment process may be a time before the start time of the next antimicrobial treatment process, may be the same time as the start time of the next antimicrobial treatment process, or may be a time after the start time of the next antimicrobial treatment process. Preferably, the ion treatment process is executed throughout the period after execution of the antimicrobial treatment process to before execution of the next antimicrobial treatment process. More preferably, the start time of the ion treatment process is a time before the end time of the first antimicrobial treatment process.

As described above with reference to FIG. 1, according to the first embodiment, the start time of the ion treatment process is included in the period from the start time of the first antimicrobial treatment process to before the start time of the next antimicrobial treatment process. Furthermore, the end time of the ion treatment process is a time after the end time of the first antimicrobial treatment process. In other words, ions are delivered to the workspace 14 during the period after execution of the first antimicrobial treatment process to before execution of the next antimicrobial treatment process. As a result, ions are present in the workspace 14 during the period after execution of the first antimicrobial treatment process to before execution of the next antimicrobial treatment process, making it possible to favorably maintain a hygienic environment in the workspace 14.

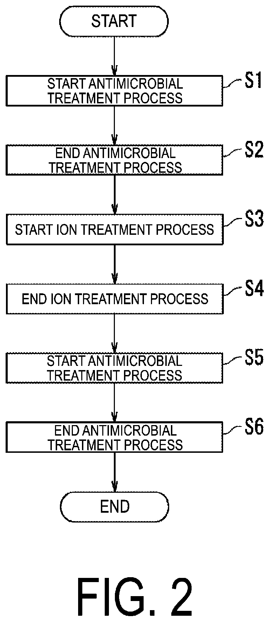

Next, an antimicrobial method according to the first embodiment will be described with reference to FIG. 2. FIG. 2 is a flowchart illustrating the antimicrobial method according to the first embodiment. As illustrated in FIG. 2, the antimicrobial method includes step S1 to step S6. The antimicrobial method is executed by the antimicrobial system 100.

First, in step S1, the antimicrobial unit 60 starts an antimicrobial treatment process of delivering ozone (O₃) toward the workspace 14 under control of the control unit 80. As a result, ozone (O₃) is present in the workspace 14.

Next, in step S2, the antimicrobial unit 60 ends the antimicrobial treatment process of delivering ozone (O₃) toward the workspace 14 under control of the control unit 80.

Next, in step S3, the ion unit 70 starts the ion treatment process of delivering ions toward the workspace 14 under control of the control unit 80. As a result, ions are present in the workspace 14.

Next, in step S4, the ion unit 70 ends the ion treatment process of delivering ions toward the workspace 14 under control of the control unit 80.

Next, in step S5, the antimicrobial unit 60 starts an antimicrobial treatment process of delivering ozone (O₃) toward the workspace 14 under control of the control unit 80. As a result, ozone (O₃) is present in the workspace 14.

Next, in step S6, the antimicrobial unit 60 ends the antimicrobial treatment process of delivering ozone (O₃) toward the workspace 14 under control of the control unit 80. With this, the antimicrobial method ends.

As described above with reference to FIG. 2, according to the first embodiment, two antimicrobial treatment processes are executed at a predetermined interval. Furthermore, ions are delivered to the workspace 14 during the period after execution of the antimicrobial treatment process to before execution of the next antimicrobial treatment process. As a result, ions are present in the workspace 14 during the period after execution of the antimicrobial treatment process to before execution of the next antimicrobial treatment process, making it possible to favorably maintain a hygienic environment in the workspace 14.

The ion unit 70 will now be described. Specifically, the ion unit 70 includes an ion generation unit and a delivery unit.

Specifically, the ion generation unit includes a pair of electric discharge electrodes (not illustrated). In particular, a positive voltage is applied to one of the pair of electric discharge electrodes. When the positive voltage is applied to the electric discharge electrode, a water molecule (H₂O) in the air is electrically decomposed by electric discharge, and a hydrogen ion H⁺ is mainly generated. Then, the water molecules (H₂O) in the air condense around the generated hydrogen ion H⁺, and a stable cluster ion H⁺(H₂O)ₘ having a positive charge is formed. m is a natural number. The stable cluster ion H⁺(H₂O)ₘ having a positive charge is an example of a positive ion. Note that generation of the cluster ion H⁺(H₂O)ₘ can be confirmed by time-of-flight mass spectrometry.

On the other hand, a negative voltage is applied to the other of the pair of electric discharge electrodes. When the negative voltage is applied to the electric discharge electrode, an oxygen molecule (O₂) in the air is ionized by electric discharge, and an oxygen molecular ion (superoxide ion) O₂⁻ is mainly generated. Then, the water molecules (H₂O) in the air condense around the generated oxygen molecular ion (superoxide ion) O₂⁻, and a stable cluster ion O₂⁻(H₂O)ₙ having a negative charge is formed. n is a natural number. The stable cluster ion O₂⁻(H₂O)ₙ having a negative charge is an example of a negative ion. Note that generation of the cluster ion H⁺(H₂O)ₘ can be confirmed by time-of-flight mass spectrometry.

The delivery unit delivers the cluster ion H⁺(H₂O)ₘ and the cluster ion O₂⁻(H₂O)ₙ to the workspace 14. The delivery unit is, for example, a fan. The fan rotates. The fan generates an air current by rotating. The air current generated by the delivery unit includes the cluster ion H⁺(H₂O)ₘ and the cluster ion O₂⁻(H₂O)ₙ, and the delivery unit can deliver the cluster ion H⁺(H₂O)ₘ and the cluster ion O₂⁻(H₂O)ₙ toward the workspace 14. Specifically, an installation position and an airflow direction of the ion unit 70 are set so that the air current generated by the delivery unit reaches every corner of the workspace 14. As a result, the cluster ion H⁺(H₂O)ₘ and the cluster ion O₂⁻(H₂O)ₙ are diffused throughout the workspace 14.

For example, when the cluster ion H⁺(H₂O)ₘ and the cluster ion O₂⁻(H₂O)ₙ are released into the air at the same time, the cluster ion H⁺(H₂O)ₘ and the cluster ion O₂⁻(H₂O)ₙ surround the surface of the microorganism. Then, the cluster ion H⁺(H₂O)ₘ and the cluster ion O₂⁻(H₂O)ₙ are momentarily bonded together, and [ • OH] (hydroxyl radical) is generated on the surface of the microorganism. The [ • OH] is an active species having high oxidizability, and thus inactivates the microorganism, making it possible to reduce the life activity of the microorganism.

Specifically, the ion unit 70 preferably supplies the cluster ion H⁺(H₂O)ₘ and the cluster ion O₂⁻(H₂O)ₙ so that concentrations of the cluster ion H⁺(H₂O)ₘ and the cluster ion O₂⁻(H₂O)ₙ in the workspace 14 are each 7000 pcs/cm³ or greater. As long as the concentrations of the cluster ion H⁺(H₂O)ₘ and the cluster ion O₂⁻(H₂O)ₙ are each 7000 pcs/cm³ or greater, the growth and the proliferation of bacteria, viruses, and the like can be reduced.

Furthermore, upper limits of the concentrations of the cluster ion H⁺(H₂O)ₘ and the cluster ion O₂⁻(H₂O)ₙ differ depending on the cells to be cultured. Thus, before the start of cell culturing, the concentrations of the cluster ion H⁺(H₂O)ₘ and the cluster ion O₂⁻(H₂O)ₙ in the workspace 14 may be set depending on the type of cell to be cultured. Specifically, the ion unit 70 preferably supplies the cluster ion H⁺(H₂O)ₘ and the cluster ion O₂⁻(H₂O)ₙ so that the concentrations of the cluster ion H⁺(H₂O)ₘ and the cluster ion O₂⁻(H₂O)ₙ in the workspace 14 are each 1 million pcs/cm³ or less. As long as the concentrations of the cluster ion H⁺(H₂O)ₘ and the cluster ion O₂⁻(H₂O)ₙ are each 1 million pcs/cm³ or less, the cluster ion H⁺(H₂O)ₘ and the cluster ion O₂⁻(H₂O)ₙ can function as antimicrobials without adversely affecting cells.

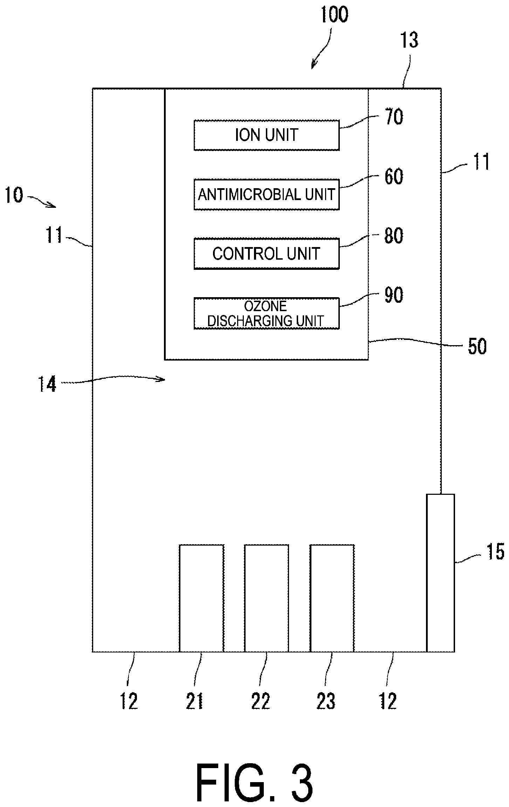

Next, the antimicrobial method according to the first embodiment will be described with reference to FIG. 3. FIG. 3 is a diagram illustrating the configuration of the antimicrobial system 100 according to the first embodiment. As illustrated in FIG. 3, the antimicrobial device 50 further includes an ozone discharging unit 90.

The ozone discharging unit 90 discharges ozone (O₃) from the workspace 14. Specifically, the ozone discharging unit 90 is a fan. The fan rotates. The fan generates an air current by rotating.

The control unit 80 switches the ozone discharging unit 90 on and off. Specifically, the control unit 80 turns on the ozone discharging unit 90 during a predetermined period after the first period. In other words, the control unit 80 executes a discharge treatment process of discharging ozone (O₃) during the predetermined period after the first period. As a result, ozone (O₃) is not present in the workspace 14. Thus, a person can be present in the workspace 14. Specifically, the concentration of ozone (O₃) in the workspace 14 is measured by using an ozone detector or the like, and a worker enters the workspace 14 after the concentration of ozone (O₃) is equal to or less than a safe standard value.

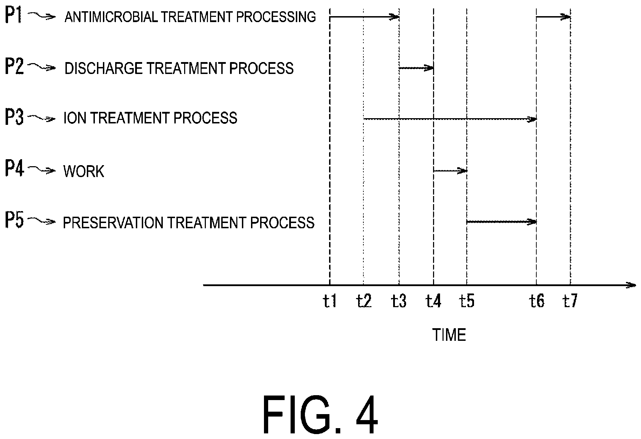

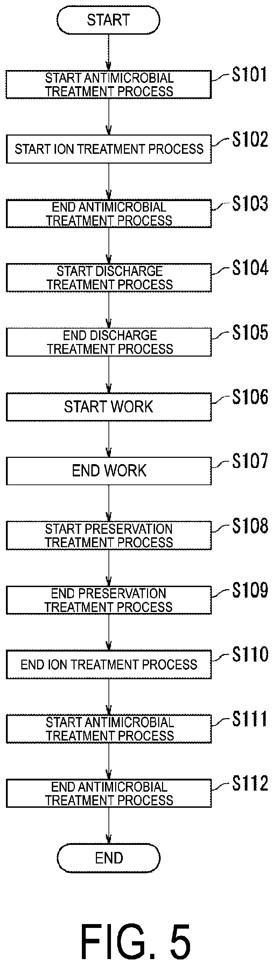

Next, the antimicrobial method according to the first embodiment will be described with reference to FIG. 4 and FIG. 5. FIG. 4 is a timetable illustrating the antimicrobial method according to the first embodiment. As illustrated in FIG. 4, the antimicrobial method includes an antimicrobial treatment process P1, a discharge treatment process P2, an ion treatment process P3, work P4, and a preservation treatment process P5. The antimicrobial method is executed by the antimicrobial system 100.

In the work P4, cell culture related work is performed by the worker in the workspace 14. The work P4 is performed in periods other than the first period. In other words, the worker is not present in the workspace 14 during the first period. As a result, the worker is not adversely affected by ozone (O₃).

In the preservation treatment process P5, cells of a multicellular organism related to regenerative medicine are preserved and cultured in the workspace 14. The preservation treatment process P5 is performed in periods other than the first period. In other words, cells are not present in the workspace 14 during the first period. As a result, the cells are not adversely affected by ozone (O₃).

FIG. 5 is a flowchart illustrating the antimicrobial method according to the first embodiment. The antimicrobial method includes step S101 to step S112.

As illustrated in FIG. 4 and FIG. 5, the antimicrobial treatment process P1 is started at time t1. Specifically, in step S101, the antimicrobial unit 60 starts delivery of ozone (O)₃ toward the workspace 14 under control of the control unit 80. As a result, ozone (O₃) is present in the workspace 14.

Next, the ion treatment process P3 is started at time t2. Specifically, in step S102, the ion unit 70 starts delivery of the cluster ion H⁺(H₂O)ₘ and the cluster ion O₂⁻(H₂O)ₙ toward the workspace 14 under control of the control unit 80. As a result, the cluster ion H⁺(H₂O)ₘ and the cluster ion O₂⁻(H₂O)ₙ are present in the workspace 14.

Next, the antimicrobial treatment process P1 is ended at time t3. Specifically, in step S103, the antimicrobial unit 60 ends delivery of ozone (O₃) toward the workspace 14 under control of the control unit 80.

Next, the discharge treatment process P2 is started at time t3. Specifically, in step S104, the ozone discharging unit 90 starts discharging ozone (O₃) from the workspace 14 under control of the control unit 80.

Next, the discharge treatment process P2 is ended at time t4. Specifically, in step S105, the ozone discharging unit 90 ends discharge of ozone (O₃) from the workspace 14 under control of the control unit 80. As a result, ozone (O₃) is not present in the workspace 14.

Next, the work P4 is started at time t4. Specifically, in step S106, the worker starts performing the work P4 related to cell culture in the workspace 14. Specifically, the worker enters the workspace 14 by opening and closing the door 15. The worker along with the cluster ion H⁺(H₂O)ₘ and the cluster ion O₂⁻(H₂O)ₙ are present in the workspace 14.

Next, the work P4 is ended at time t5. Specifically, in step S107, the worker ends performing the work P4 related to cell culture in the workspace 14. Specifically, the worker along with the cluster ion H⁺(H₂O)ₘ and the cluster ion O₂⁻(H₂O)ₙ are present in the workspace 14. The worker exits the workspace 14 by opening and closing the door 15.

Next, the preservation treatment process P5 is started at time t5. Specifically, in step S108, the culturing of cells in the workspace 14 is started. Specifically, the cells along with the cluster ion H⁺(H₂O)ₘ and the cluster ion O₂⁻(H₂O)ₙ are present in the workspace 14.

Next, the preservation treatment process P5 is ended at time t6. Specifically, in step S109, the culturing of cells in the workspace 14 is ended. Specifically, the cells along with the cluster ion H⁺(H₂O)ₘ and the cluster ion O₂⁻(H₂O)ₙ are present in the workspace 14.

Next, the ion treatment process P3 is ended at time t6. Specifically, in step S110, the ion unit 70 ends delivery of the cluster ion H⁺(H₂O)ₘ and the cluster ion O₂⁻(H₂O)ₙ toward the workspace 14 under control of the control unit 80. Specifically, the worker enters and exits the workspace 14 in order to remove the cultured cells from the workspace 14 by opening and closing the door 15.

Next, the antimicrobial treatment process P1 is started at time t6. Specifically, in step S 111, the antimicrobial unit 60 starts delivery of ozone (O₃) toward the workspace 14 under control of the control unit 80. As a result, ozone (O₃) is present in the workspace 14.

Next, the antimicrobial treatment process P1 is ended at time t7. Specifically, in step S 112, the antimicrobial unit 60 ends delivery of ozone (O₃) toward the workspace 14 under control of the control unit 80. With this, the antimicrobial method ends.

As described above with reference to FIG. 5, according to the first embodiment, when the ion treatment process P3 is executed during the period after execution of the antimicrobial treatment process P1 and before execution of the next antimicrobial treatment process P1, the worker performs the work P4 related to cell culture and performs the preservation treatment process P5. As a result, the worker can perform the work P4 and perform the preservation treatment process P5 in the workspace 14 in which a hygienic environment is favorably maintained. Specifically, after the antimicrobial treatment process P1, the ion treatment process P3 is continually performed during a work phase in which the worker enters and performs work. As a result, even in a case in which a microorganism contaminates the workspace 14 during entry of the worker, it is possible to kill the microorganism or suppress the growth and the proliferation of the microorganism and favorably maintain the hygienic environment of the workspace 14.

### Second Embodiment

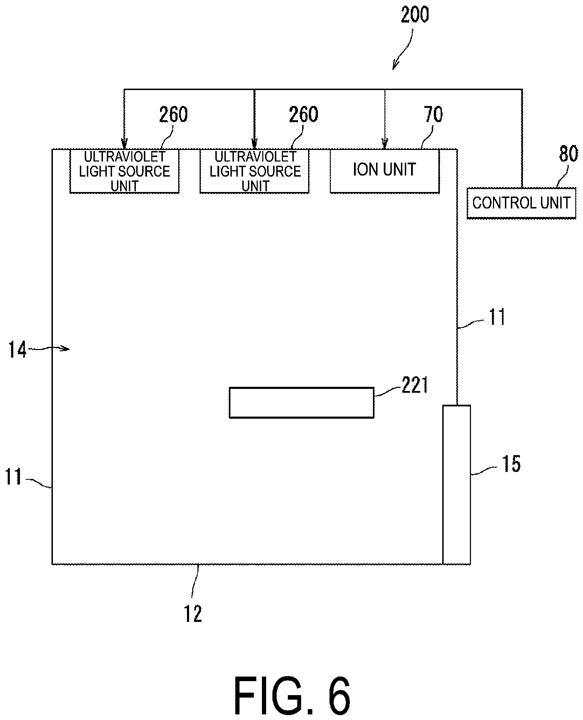

Next, an antimicrobial system 200 according to a second embodiment of the present invention will be described with reference to FIG. 6. The antimicrobial system 200 of the second embodiment differs from that in the first embodiment in being an operating room. Furthermore, the antimicrobial system 200 of the second embodiment differs from that in the first embodiment in including a plurality of ultraviolet light source units 260. Hereinafter, matters of the second embodiment that are different from those of the first embodiment will be described, and description of parts that are duplicates of the first embodiment will be omitted.

FIG. 6 is a diagram illustrating a configuration of the antimicrobial system 200 according to the second embodiment of the present invention. As illustrated in FIG. 6, the antimicrobial system 200 is an operating room for a physician to perform surgery on a patient, for example.

In the workspace 14, for example, surgical equipment 221 is disposed.

The antimicrobial device 50 includes the plurality of ultraviolet light source units 260. Each of the plurality of ultraviolet light source units 260 includes an ultraviolet light source. The ultraviolet light source emits ultraviolet light. The ultraviolet light is an example of light having an antimicrobial effect. Specifically, installation positions and the number of the ultraviolet light source units 260 are set so that ultraviolet light reaches every corner of the workspace 14. As a result, the entire workspace 14 is irradiated with ultraviolet light.

When a microorganism is irradiated with ultraviolet light, ultraviolet light can inactivate the microorganism and reduce the life activity of the microorganism. Note that ultraviolet light has very high energy and thus may adversely affect not only microorganisms, but also humans and cells when humans and cells are irradiated therewith.

Specifically, the control unit 80 turns on the ultraviolet light source units 260 in the first period and turns off the ultraviolet light source units 260 in periods other than the first period. In other words, the control unit 80 executes an antimicrobial treatment process of irradiating the workspace 14 with ultraviolet light from the ultraviolet light source units 260 during the first period.

As described above with reference to FIG. 6, according to the second embodiment, the antimicrobial treatment process of irradiating the workspace 14 with ultraviolet light is executed at a predetermined interval (for example, 1 day or 1 week). Furthermore, the cluster ion H⁺(H₂O)ₘ and the cluster ion O₂⁻(H₂O)ₙ are delivered to the workspace 14 during the period after execution of the antimicrobial treatment process to before execution of the next antimicrobial treatment process. As a result, the cluster ion H⁺(H₂O)ₘ and the cluster ion O₂⁻(H₂O)ₙ are present in the workspace 14, making it possible to suppress the presence of microorganisms in the workspace 14. Furthermore, in the case of irradiation with ultraviolet light, there is no residue of a substance such as ozone (O₃), and thus it is possible enter the workspace 14 from the moment the ultraviolet light source units 260 are turned off.

### Third Embodiment

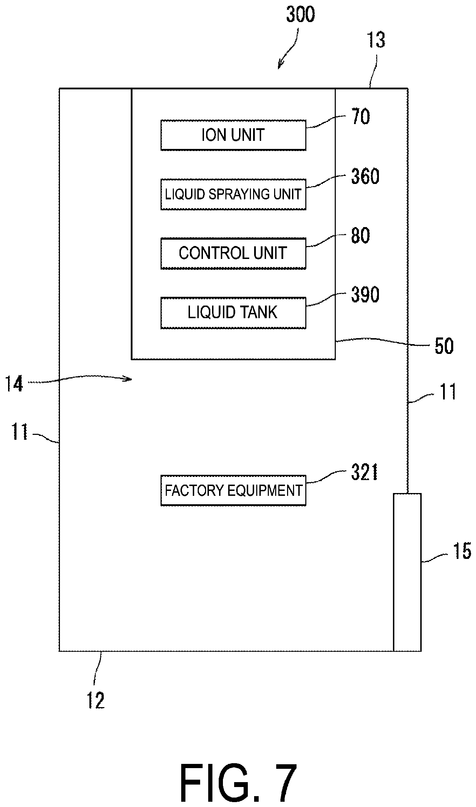

Next, an antimicrobial system 300 according to a third embodiment of the present invention will be described with reference to FIG. 7. Furthermore, the antimicrobial system 300 of the third embodiment differs from that in the first embodiment in being a food factory. Furthermore, the antimicrobial device 50 according to the third embodiment differs from that in the first embodiment in including a liquid spraying unit 360 and a liquid tank 390. Hereinafter, matters of the third embodiment that are different from those of the first embodiment will be described, and description of parts that are duplicates of the first embodiment will be omitted.

FIG. 7 is a diagram illustrating a configuration of the antimicrobial system 300 according to the third embodiment of the present invention. As illustrated in FIG. 7, the antimicrobial system 300 is, for example, a food factory for a worker to process food with yeast.

In the workspace 14, for example, factory equipment 321 is disposed.

The antimicrobial device 50 includes the liquid spraying unit 360 and the liquid tank 390. The liquid spraying unit 360 is, for example, a device for spraying ethanol from the liquid tank 390 as liquid particles. Ethanol is an example of liquid particles having an antimicrobial effect.

Specifically, installation positions and the number of the liquid spraying units 360 are set so that the liquid particles reach every corner of the workspace 14. As a result, ethanol diffuses throughout the workspace 14.

Ethanol surrounds the surface of the microorganism when released into the air. Ethanol can inactivate the microorganism and reduce the life activity of microorganism. Note that ethanol may adversely affect, not only the microorganism, but humans and yeast when released to humans and yeast.

Specifically, the control unit 80 turns on the liquid spraying unit 360 in the first period and turns off the liquid spraying unit 360 in periods other than the first period. In other words, the control unit 80 executes an antimicrobial treatment process of spraying the workspace 14 with ethanol from the liquid spraying unit 360 during the first period.

As described above with reference to FIG. 7, according to the third embodiment, the antimicrobial treatment process of spraying the workspace 14 with ethanol is executed at a predetermined interval (for example, 1 day or 1 week). Furthermore, the cluster ion H⁺(H₂O)ₘ and the cluster ion O₂⁻(H₂O)ₙ are delivered to the workspace 14 during the period after execution of the antimicrobial treatment process to before execution of the next antimicrobial treatment process. As a result, the cluster ion H⁺(H₂O)ₘ and the cluster ion O₂⁻(H₂O)ₙ are present in the workspace 14, making it possible to favorably maintain a hygienic environment in the workspace 14.

### Fourth Embodiment

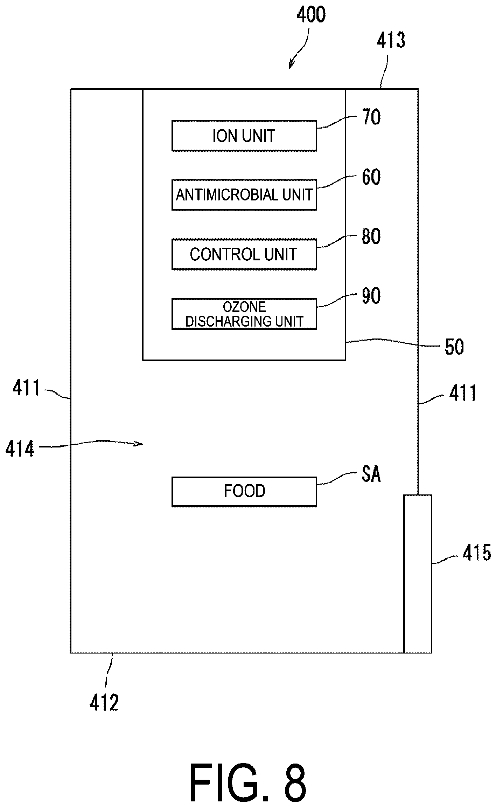

Next, an antimicrobial system 400 according to a fourth embodiment of the present invention will be described with reference to FIG. 8. Furthermore, the antimicrobial system 400 of the fourth embodiment differs from that in the first embodiment in being a container. Hereinafter, matters of the fourth embodiment that are different from those of the first embodiment will be described, and description of parts that are duplicates of the first embodiment will be omitted.

FIG. 8 is a diagram illustrating a configuration of the antimicrobial system 400 according to the fourth embodiment of the present invention. As illustrated in FIG. 8, the antimicrobial system 400 is, for example, a container for containing a food SA.

Specifically, the antimicrobial system 400 includes a container 410 and the antimicrobial device 50.

The container 410 includes a plurality of wall surfaces 411, a bottom wall 412, and an upper wall 413. An accommodation space 414 is defined by the plurality of wall surfaces 411, the bottom wall 412, and the upper wall 413. The accommodation space 414 is an example of a target space. The accommodation space 414 is a space for accommodating food. A door 415 is provided on at least one wall surface 411 of the plurality of wall surfaces 411. The food SA is put into and taken out from the accommodation space 414 by the door 415 being opened and closed.

The antimicrobial device 50 is disposed on at least one location of the plurality of wall surfaces 411, the bottom wall 412, and the upper wall 413. Specifically, the antimicrobial device 50 is disposed on the upper wall 413.

As described above with reference to FIG. 8, according to the fourth embodiment, the antimicrobial treatment process of delivering ozone (O₃) toward the accommodation space 414 is executed at a predetermined interval (for example, 1 day or 1 week). Furthermore, the cluster ion H⁺(H₂O)ₘ and the cluster ion O₂⁻(H₂O)ₙ are delivered to the accommodation space 414 during the period after execution of the antimicrobial treatment process to before execution of the next antimicrobial treatment process. As a result, the cluster ion H⁺(H₂O)ₘ and the cluster ion O₂⁻(H₂O)ₙ are present in the accommodation space 414, making it possible to favorably maintain a hygienic environment in the accommodation space 414.

The embodiments of the present invention have been described above with reference to the accompanying drawings. However, the present invention is not limited to the embodiments described above, and the present invention can be implemented in various modes without departing from the gist thereof. Further, various inventions can be formed by appropriately combining a plurality of components disclosed in the embodiments described above. For example, some components may be removed from all of the components described in the embodiments. Furthermore, the components across different embodiments may be appropriately combined. For ease of understanding, the drawings schematically illustrate each component as a main constituent, and the thickness, length, number, spacing, and the like of each component illustrated are different from the actual thickness, length, number, and spacing for convenience of drawing preparation. Further, the speed, material, shape, dimensions, and the like of each component illustrated in the embodiments described above are one example and are not particularly limited, and various modifications can be made within a range that does not substantially deviate from the configuration of the present invention.
(1) As described with reference to FIG. 1 to FIG. 5, in the first embodiment, the ion treatment process is executed once per period after execution of the antimicrobial treatment process and before execution of the next antimicrobial treatment process, but the present invention is not limited thereto. The ion treatment may be executed a plurality of times in the period after execution of the antimicrobial treatment process to before execution of the next antimicrobial treatment process. In other words, the control unit 80 may repeatedly turn the ion unit 70 on and off during the period after execution of the antimicrobial treatment process to before execution of the next antimicrobial treatment process.
(2) In the first embodiment, the antimicrobial unit 60 delivers ozone (O₃) toward the workspace 14, but the present invention is not limited thereto. The antimicrobial unit 60 may deliver hydrogen peroxide gas toward the workspace 14.
(3) In the third embodiment, the liquid spraying unit 360 is a device that sprays ethanol as liquid particles, but the present invention is not limited thereto. The liquid spraying unit 360 may be a device for spraying formalin as liquid particles.

### Industrial Applicability

The present invention is applicable in the fields of an antimicrobial method and an antimicrobial device.

### Reference Signs List

14 Target space
50 Antimicrobial device
60 Antimicrobial unit
70 Ion unit
80 Control unit

## Claims

1. An antimicrobial method comprising:
delivering, at least twice, at least one of light having an antimicrobial effect, a gas having an antimicrobial effect, or liquid particles having an antimicrobial effect toward a target space; and
delivering ions toward the target space,
wherein a start time of the delivering the ions is included in a period from a start time of a first instance of the delivering the at least one of the light having the antimicrobial effect, the gas having the antimicrobial effect, or the liquid particles having the antimicrobial effect to before a start time of a second instance of the delivering the at least one of the light having the antimicrobial effect, the gas having the antimicrobial effect, or the liquid particles having the antimicrobial effect, and
an end time of the delivering the ions is after an end time of the first instance of the delivering the at least one of the light having the antimicrobial effect, the gas having the antimicrobial effect, or the liquid particles having the antimicrobial effect.

2. The antimicrobial method according to claim 1,
wherein each of the antimicrobial effect of the light, the antimicrobial effect of the gas, and the antimicrobial effect of the liquid particles is stronger than an antimicrobial effect of the ions, and
during the delivering the at least one of the light having the antimicrobial effect, the gas having the antimicrobial effect, or the liquid particles having the antimicrobial effect, a worker is not present in the target space.

3. The antimicrobial method according to claim 2,
wherein work by the worker is performed in the target space during the delivering the ions.

4. The antimicrobial method according to any one of claims 1 to 3,
wherein the start time of the delivering the ions is before the end time of the delivering the at least one of the light having the antimicrobial effect, the gas having the antimicrobial effect, or the liquid particles having the antimicrobial effect.

5. The antimicrobial method according to any one of claims 1 to 4, further comprising:
after delivering the gas or the liquid particles in the delivering the at least one of the light having the antimicrobial effect, the gas having the antimicrobial effect, or the liquid particles having the antimicrobial effect, discharging the gas or the liquid particles from the target space.

6. An antimicrobial device comprising:
an antimicrobial unit configured to deliver at least one of light having an antimicrobial effect, a gas having an antimicrobial effect, or liquid particles having an antimicrobial effect toward a target space;
an ion unit configured to deliver ions toward the target space; and
a control unit configured to control the antimicrobial unit and the ion unit.
